# EUROPÄISCHE PATENTSCHRIFT

(11) **EP 4 299 022 B1**
(45) Veröffentlichungstag und Bekanntmachung des Hinweises auf die Patenterteilung: **05.03.2025**
(21) Anmeldenummer: 23182146.3
(22) Anmeldetag: 28.06.2023
(51) Int. Cl.: A61B 18/12, H02M 7/539, A61B 18/14

(54) **ELEKTROCHIRURGISCHER GENERATOR MIT VERBESSERTER INVERTERSTEUERUNG UND ENTSPRECHENDES VERFAHREN**
ELECTROSURGICAL GENERATOR WITH IMPROVED INVERTER CONTROL AND RELATED METHOD
GÉNÉRATEUR ÉLECTROCHIRURGICAL AVEC COMMANDE D'ONDULEUR AMÉLIORÉE ET PROCÉDÉ CORRESPONDANT

(30) Priorität: 29.06.2022 US 202263356631 P
(43) Veröffentlichungstag der Anmeldung: 03.01.2024
(73) Patentinhaber: Olympus Winter & Ibe GmbH, 22045 Hamburg (DE)
(72) Erfinder: Janich, Fabian, 14469 Potsdam (DE); Dijkstra, Jelle, 12205 Berlin (DE); Behrendt, Raphael, 12103 Berlin (DE)
(74) Vertreter: Glawe, Delfs, Moll

(56) Entgegenhaltungen:
- EP-A1- 3 248 560
- EP-A1- 4 140 426
- EP-A1- 4 140 427
- US-A1- 2021 361 340
- US-A1- 2022 069 733
- ZHAO CHONGWEN ET AL: "A single-phase dual frequency inverter based on multi-frequency selective harmonic elimination", 2016 IEEE APPLIED POWER ELECTRONICS CONFERENCE AND EXPOSITION (APEC), IEEE, 20 March 2016 (2016-03-20), pages 3577 - 3584, XP032899295, ISBN: 978-1-4673-8393-6, [retrieved on 20160510], DOI: 10.1109/APEC.2016.7468383

## Beschreibung

Die Erfindung betrifft einen elektrochirurgischen Generator zur Abgabe einer hochfrequenten Wechselspannung an ein elektrochirurgisches Instrument und ein entsprechendes Verfahren. Der elektrochirurgische Generator umfasst eine Steuereinheit und einen Inverter für Hochspannung, der eine hochfrequente Wechselspannung mit variabler Frequenz und Amplitude erzeugt, die einer Ausgangsbuchse zum Anschluss des elektrochirurgischen Instruments zugeführt wird

Elektrochirurgische Generatoren weisen in der Regel eine recht komplexe Funktionalität auf, die eine große Anzahl von Funktionen umfasst. Dadurch werden die chirurgischen Generatoren in die Lage versetzt, verschiedene Anwendungen zu bewältigen. Diese Funktionen unterscheiden sich in der Art und Weise, wie sie das elektrochirurgische Instrument mit Energie versorgen. Die verschiedenen Möglichkeiten, das elektrochirurgische Instrument mit Energie zu versorgen, werden üblicherweise als "Modes" bezeichnet. Wenn lediglich Gewebe geschnitten werden soll, wird ein Schneidmodus gewählt, der das elektrochirurgische Instrument kontinuierlich mit einer mittleren Spannung versorgt. Wenn Koagulation wichtiger ist, wird ein anderer Modus gewählt, z. B. ein Sprüh- oder Koagulationsmodus, bei dem eine relativ hohe Spannung mit einem niedrigen Tastverhältnis verwendet wird. Es gibt noch viele weitere verschiedene Modi für andere Anwendungsarten.

Gegenwärtig verfügen elektrochirurgische Generatoren über Hochspannungsgeneratoren, die meist mit Hilfe von Resonanzwandlern erzeugt werden. Dies ist eine zuverlässige und bewährte Technologie. Sie hat jedoch Nachteile dahingehend, dass die Frequenz nicht kontrolliert steuerbar ist und die Amplitude nur durch Änderung der Spannung der Gleichstromversorgung für den Resonanzwandler eingestellt werden kann. Jegliche Änderungen der Frequenz und/oder der Amplitude sind ziemlich unhandlich.

Um die Regelbarkeit zu verbessern, ist es bekannt, einen elektrochirurgischen Generator mit einem Inverter in Form einer H-Brücke zu versehen (EP 3 248 560 A1). Um die Bildung übermäßiger Oberschwingungen zu vermeiden, müssen jedoch spezielle Steuermethoden eingesetzt werden.

Darüber hinaus hat die Anmelderin elektrochirurgische Generatoren entwickelt, die aus Multilevel-Invertern in einer kaskadierten Topologie bestehen. Dadurch konnte die Einstellbarkeit des Ausgangssignals erheblich verbessert werden. Schnelle Änderungen von Amplitude und Frequenz sind möglich. Weiterhin sind prinzipiell beliebige Kurvenformen der Ausgangsspannung möglich. Dazu ist jedoch ein spezieller Hochleistungscontroller, z. B. ein FPGA, erforderlich, der in der Lage ist, die Stromventile des Wechselrichters mit einer wesentlich höheren Frequenz anzusteuern als die Ausgangsfrequenz der erzeugten Hochspannung. Typische Ansteuerungsfrequenzen sind 200 MHz für eine Ausgangsspannung, die eine Frequenz von 400 kHz aufweist. Das ist machbar, aber die Bereitstellung des Referenzsignals für eine so hohe Ansteuerungsfrequenz erfordert viele Datenpunkte in rascher Folge, was wiederum Hochgeschwindigkeits-Controller erfordert, die kostspielig sind.

Es ist eine Aufgabe der Erfindung, einen elektrochirurgischen Generator und eine verbesserte Invertersteuerung bereitzustellen, die diesen Nachteil verringert.

Die erfindungsgemäße Lösung liegt in den Merkmalen der unabhängigen Ansprüche. Vorteilhafte Ausführungsformen sind Gegenstand der abhängigen Ansprüche.

Bei einem elektrochirurgischen Generator zur Ausgabe einer Hochfrequenz-Wechselspannung an ein elektrochirurgisches Instrument, umfassend eine Steuereinheit und einen Inverter für Hochspannung, der eine Hochfrequenz-Wechselspannung mit variabler Frequenz und Amplitude erzeugt, die einer Ausgangsbuchse zum Anschluss des elektrochirurgischen Instruments zugeführt wird, ist erfindungsgemäß vorgesehen, dass der Inverter ein Multilevel-Inverter ist, der durch ein Referenzsignal gesteuert wird, das die Form der erzeugten Hochfrequenz-Wechselspannung definiert, wobei das Referenzsignal durch ein Datagramm definiert ist, wobei das Datagramm eine endliche Reihe von aufeinanderfolgenden Amplituden- und Frequenzdatenvektoren für eine definierte Anzahl von Perioden umfasst.

Zunächst sollen einige Begriffe erklärt werden:
Ein Multilevel-Inverter ist ein Wechselrichter, der in der Lage ist, eine Ausgangsspannung mit verschiedenen Pegeln abzugeben, im Gegensatz zu einem Ausgang mit lediglich Ein/Aus mit positiver und/oder negativer Polarität. Typische Topologien sind u. a. kaskadierte H-Brücken, neutral geklemmte und fliegende Kondensatoren.

Unter einem Datagramm versteht man ein Datenpaket mit einer endlichen Länge. Es ist typischerweise klein bis max. 256 Byte, insbesondere 50 Byte oder weniger. Es kann optional eine Quell- und Zielidentifikation enthalten, was aber nicht notwendig ist.

Der Begriff "Periode" wird hier als Bezeichnung für die Zeit verwendet, die benötigt wird, um eine volle Schwingung einer Wellenform abzuschließen. Typischerweise bezieht sich dies auf die Wellenform der Hochfrequenz-Wechselspannung, wie sie vom Inverter erzeugt wird.

Die Steuerung des Inverters und die Bereitstellung seines Referenzsignals gemäß der vorliegenden Erfindung macht einen teuren Hochgeschwindigkeits-Controller, wie z. B. einen FPGA, überflüssig. Die Erfindung bietet einen anderen Ansatz für die Bildung des Referenzsignals, insbesondere die Bildung auf der Grundlage eines Datagramms, das eine flexible und präzise Beschreibung des zu erzeugenden Referenzsignals liefert. Zu diesem Zweck wurde ein spezielles Datenformat entwickelt, das eine endliche Reihe von aufeinanderfolgenden Amplituden- und Frequenzdatenvektoren für eine bestimmte Anzahl von Perioden aufweist. Das Datagramm beschreibt das zu erzeugende Referenzsignal mit einem Minimum an Informationen.

Elektrochirurgische Generatoren stellen ihre hochfrequente Wechselspannung in verschiedenen Modi zur Verfügung. Ein einfacher Modus wird als kontinuierliche oszillierende Spannung bereitgestellt, z. B. für einen "PureCut"-Modus. Darüber hinaus gibt es mehrere komplexere Modi, die so konfiguriert sind, dass die Ausgangsspannung in Bezug auf Frequenz, Amplitude, Tastverhältnis und/oder Scheitelfaktor über die Zeit variiert. Ein Beispiel ist ein "SprayCoag"-Modus, bei dem eine sehr hohe Spannung in Form einer Spitze für eine sehr kurze Zeit anliegt, z. B. für eine oder zwei Oszillationen, gefolgt von einer sehr viel längeren Pause mit sehr niedriger oder keiner Spannung, wobei die Dauer der Pause mindestens eine Dimension (10-mal) länger ist als die Spitze, z. B. 20-mal länger.

Um die Ausgangsspannung gemäß ziemlich komplexen Modi erzeugen zu können, kann die Bereitstellung eines Referenzsignals für einen Multilevel-Inverter recht datenintensiv sein. Ein Multilevel- Inverter, der eine hochfrequente Ausgangswechselspannung von bis zu 500 kHz erzeugt, wird in der Regel mit einer viel höheren Frequenz im Bereich von typischerweise etwa 200 MHz angesteuert. Je nach Anzahl der Stufen kann für jeden Datenpunkt ein halbes, ein oder sogar zwei Byte erforderlich sein. Dies führt zu einem recht hohen Bandbreitenbedarf, der anspruchsvoll und kostspielig ist.

Im Rahmen der Erfindung wurde erkannt, dass die Datenmenge und damit die benötigte Bandbreite drastisch reduziert werden kann, indem ein Datagramm bereitgestellt wird, das beschreibende Daten für die gewünschte hochfrequente sinusförmige Kurve der Ausgangsspannung enthält anstatt eines umfangreichen Stroms von Datenpunkten. Dies wird recht deutlich, wenn die gewünschte Ausgangsspannung eine kontinuierliche Spannung ist, bei der das Datagramm nur eine zweistellige Anzahl von Bits benötigt. Aber überraschenderweise benötigen auch komplexere Modi nur eine geringe Datenmenge, wenn das erfindungsgemäße Datagramm verwendet wird, nämlich eine endliche Reihe von aufeinanderfolgenden Amplituden- und Frequenzdatenvektoren für eine bestimmte Anzahl von Perioden. Auf diese Weise kann eine Vielzahl von Modi mit einem Minimum an Daten beschrieben werden, die nur einige Dutzend Bytes lang sein brauchen. Dies stellt offensichtlich eine drastische Verringerung des Bandbreitenbedarfs dar.

Vorzugsweise ist das Referenzsignal von einer Referenzsignalbildungseinheit gebildet. Sie ist so konfiguriert, dass sie das Referenzsignal gemäß dem Datagramm bildet. Weiter vorzugsweise umfasst die Referenzsignalbildungseinheit einen Empfänger, der zum Empfangen eines das Referenzsignal beschreibenden Datensatzes eingerichtet ist. Der reduzierte Bandbreitenbedarf der erfindungsgemäßen Datagramme ermöglicht dabei eine erhöhte Freiheit hinsichtlich der Positionierung der Referenzsignalbildungseinheit innerhalb des elektrochirurgischen Generators, ohne auf die Nähe zur Quelle des Datagramms beschränkt zu sein.

Die Erfindung ermöglicht vorzugsweise eine effiziente Erzeugung des Referenzsignals durch Verwendung eines Synthesizers. Zu diesem Zweck umfasst die Referenzsignalbildungseinheit vorzugsweise einen Decoder, der so konfiguriert ist, dass er den vom Empfänger empfangenen Datensatz dekodiert und die Datenvektoren für jede der Perioden extrahiert, einen Sequenzer, der so konfiguriert ist, dass er Signale für Amplitude und Frequenz aus dem extrahierten Datenvektor für jede Periode in einer durch die Datenfelder definierten Folge ausgibt und diese Folge wiederholt; und einen Synthesizer, der so konfiguriert ist, dass er für jeden der Datenvektoren eine Oszillationswelle mit der Amplitude und Frequenz gemäß dem jeweiligen Datenvektor bildet, wobei eine Reihe der Oszillationswellen das Referenzsignal bildet, das dem Inverter zugeführt wird. In Verbindung mit der erfindungsgemäßen Steuerung durch die Datagramme lassen sich auf einfache Weise sowohl kontinuierliche als auch nicht-kontinuierliche Referenzsignale für die Ausgangsspannung bilden.

Vorzugsweise umfasst das Datagramm eine Vielzahl von Datenfeldern, ein Datenfeld für jede Periode, wobei jedes Datenfeld umfasst einen der Datenvektoren mit Werten, die für Amplitude und Frequenz einer jeweiligen der definierten Anzahl von Perioden indikativ sind. Die Bereitstellung eines eigenen Datenfeldes, das den Datenvektor für jede der Perioden enthält, ermöglicht ein hohes Maß an Flexibilität bei der Definition einer komplexen Sequenz, wodurch die Referenzsignalbildungseinheit in die Lage versetzt wird, Referenzsignale selbst für komplexe Modi des elektrochirurgischen Generators zu bilden, wie z. B. Modi mit durch Pausen getrennten Spannungsimpulsen.

Vorteilhafterweise enthält das Datagramm ferner ein erstes zusätzliches Datenfeld, das indikativ ist für eine Polarität, insbesondere die Polarität einer ersten Halbwelle einer ersten Periode. Dadurch kann ausgewählt werden, ob das Referenzsignal mit einer positiven Halbwelle oder mit einer negativen Halbwelle beginnt, und damit kann die Polarität (positive oder negative erste Halbwelle) der Hochspannungsausgangsspannung durch den Chirurgen mit dem HF-Generator ausgewählt werden.

Vorteilhafterweise enthält das Datagramm außerdem ein zweites zusätzliches Datenfeld, das indikativ ist für die Anzahl der Perioden. Dadurch werden der Empfänger und insbesondere der Decoder und Sequenzer in die Lage versetzt, das Datagramm auf Vollständigkeit zu prüfen. Dadurch können fehlerhafte Datagramme oder Datagramme, denen Datenvektoren für einige Perioden fehlen, leicht erkannt und zurückgewiesen werden, wodurch die Betriebssicherheit erhöht wird.

Zusätzlich oder alternativ kann das Datagramm vorzugsweise zusätzliche Datenfelder enthalten, die indikativ sind für Start- und Stoppinformationen, insbesondere Start- und Stoppbytes. Dadurch werden Anfang und Ende des Datagramms eindeutig identifiziert. Damit wird die Datenqualität des Datagramms weiter verbessert. Durch die Verwendung von Start- und Stoppinformationen ist es auch möglich, Datagramme mit einer unbestimmten Anzahl von Perioden zu haben, so dass die Aufnahme zusätzlicher Perioden mit zusätzlichen Datenvektoren für komplexere Codes erleichtert wird. Dies verbessert die Fähigkeit für zukünftige Aktualisierungen. Darüber hinaus können die Start- und/oder Stoppinformationen optional die Richtung des Datenflusses bestimmen, wodurch die Daten des Datagramms ausgelesen werden, und/oder sie können Datenintegritätsdatenfelder umfassen, z. B. eine Prüfsumme wie eine CRC (zyklische Redundanzprüfsumme).

Vorzugsweise umfasst das Datagramm mindestens Datenfelder für zwei Perioden. Dadurch wird eine recht freie Wahl des Tastverhältnisses ermöglicht, was ein großer Vorteil bei der Definition komplexer Modi des elektrochirurgischen Generators ist. In einer bevorzugten Ausführungsform ist der Synthesizer zur Bildung von Halbwellenschwingungen ausgebildet, und die Perioden im Datagramm beziehen sich auf die Dauer der Halbwellenschwingung. Dabei könnte für jede Halbwelle eine individuelle Frequenz und Amplitude zugewiesen werden. Dies ermöglicht eine noch höhere Granularität bei der Definition der Spannungskurven der Moden. Außerdem erlaubt dies eine asymmetrische Formung des Referenzsignals, wodurch die Freiheit bei der Gestaltung noch komplexerer Moden weiter erhöht wird. Es ist erwähnenswert, dass aufgrund des Datenvektorkonzepts die Frequenz und/oder Amplitude der zweiten Halbwelle von der Frequenz und/oder Amplitude der ersten Halbwelle abweichen können. Dies ist ein wichtiger Aspekt der Erfindung, und insbesondere die Möglichkeit, unterschiedliche Frequenzen für die erste und zweite Halbwelle zu haben, ist ein großer Vorteil. Dementsprechend bietet die Halbwellenfähigkeit eine größere Flexibilität bei der Gestaltung einer großen Vielfalt unterschiedlicher Referenzsignale für verschiedene Modi.

Außerdem kann der Synthesizer so konfiguriert sein, dass er nur unipolare Halbwellen erzeugt, insbesondere positive Halbwellen. Ein Synthesizer, der nur Halbwellen erzeugen kann, kann vereinfacht werden im Vergleich zu einem bipolaren Synthesizer. Außerdem kann bei gleicher Bitanzahl eine höhere Quantisierung und damit eine bessere Auflösung in Bezug auf die Spannungsamplitude erreicht werden, was eine feinere Reproduktion von sinusförmigen Spannungsverläufen ermöglicht. Ein weiterer Vorteil ist, dass aufgrund der besseren Quantisierung der Anteil unerwünschter Oberwellen reduziert werden kann.

Vorzugsweise ist der Synthesizer mit einer Inversionsschaltung gekoppelt, die so konfiguriert ist, dass sie jede zweite vom Synthesizer abgegebene Halbwelle invertiert. Durch die Invertierung jeder zweiten Halbwelle kann mit dem vereinfachten Halbwellensynthesizer eine vollständige bipolare Schwingung erzeugt werden.

Vorzugsweise handelt es sich bei der Inversionsschaltung um eine schaltbare Inversionsschaltung, bei der in Abhängigkeit von einem Auswahlsignal umschaltbar ist, ob gerade oder ungerade Halbwellen invertiert werden sollen. Dabei kann durch das Auswahlsignal auf einfache Weise bestimmt werden, ob das Referenzsignal und damit die zu bildende Schwingung mit einer positiven oder einer negativen Halbwelle beginnt. Vorzugsweise wird das Auswahlsignal der Inversionsschaltung automatisch gemäß einem ersten zusätzlichen Datenfeld eingestellt, das die Polarität angibt.

Insbesondere wenn der elektrochirurgische Generator, der die Ausgangsspannung an das elektrochirurgische Instrument liefert, über einen längeren Zeitraum in Betrieb ist oder bei komplexen Betriebsarten, wie z. B. solchen, die einen Wechsel zwischen verschiedenen Betriebsarten umfassen (z. B. Wechsel zwischen CUT- und COAG-Betrieb), kann es von Zeit zu Zeit erforderlich sein, die Datenvektoren einiger, aber nicht aller Perioden des Referenzsignals zu aktualisieren. Natürlich könnte ein neues vollständiges Datagramm erstellt und gesendet werden, um von der Referenzsignalbildungseinheit empfangen und verarbeitet zu werden. Es ist jedoch noch effizienter, nicht das gesamte Datagramm, sondern nur einen reduzierten Satz (Fragment) zu senden. Vorteilhafterweise ist der Empfänger ferner so konfiguriert, dass er ein Datagrammfragment empfängt, wobei das Datagrammfragment eine eingeschränkte Anzahl der Datenfelder mit geänderten Amplituden- und Frequenzdatenvektoren und eine Kennung für die betreffende Periode umfasst, wobei die eingeschränkte Anzahl eine oder mehrere, aber weniger als die definierte Anzahl von Perioden beträgt. Dadurch wird die Referenzsignalbildungseinheit in die Lage versetzt, Amplituden- und Frequenzdatenvektoren für eine (oder einige) bestimmte Perioden selektiv zu ersetzen, ohne dass die vollständige Übertragung des gesamten Datagramms erforderlich ist. Dadurch wird die Menge der zu übertragenden Daten weiter reduziert. Außerdem ermöglicht es schnellere Aktualisierungen, was sehr wichtig ist für einen schnellen Wechsel zwischen den Betriebsarten, z. B. zwischen CUT- und COAG-Modus.

Zu diesem Zweck ist der Decoder vorzugsweise dazu ausgebildet ist die Datenvektoren und Identifikatoren zu extrahieren, und der Sequenzer ist dazu ausgebildet, selektiv für die betreffende Periode die Ausgangssignale für Amplitude und Frequenz zu ersetzen durch diejenigen, die in dem dekodierten Datagrammfragment empfangen werden. Dabei müssen nur die tatsächlich veränderten Datenvektoren ersetzt werden, was das Verfahren vereinfacht und beschleunigt.

Vorteilhafterweise ist das Datagramm so verdichtet, dass eine Bitlänge von Amplituden- und/oder Frequenzwert im Datenvektor auf die Anzahl der Pegel des Multilevel-Inverters bzw. den Frequenzbereich begrenzt ist. Dadurch kann die Übertragung von unnötig langen Daten vermieden werden, was zu noch kompakteren Datagrammen führt, die eine schnellere Übertragung und Verarbeitung ermöglichen.

Vorzugsweise ist das Datagramm kleiner als 256 Bytes, vorzugsweise kleiner als 50 Bytes.

In den meisten Fällen ist der Empfänger mit der Steuereinheit für den Empfang des Datagramms gekoppelt, wobei sich die Steuereinheit lokal am elektrochirurgischen Generator befindet. In einer vorteilhaften Ausführungsform ist es jedoch auch möglich, dass der Empfänger so konfiguriert ist, dass er das Datagramm von einer entfernten Steuereinheit empfängt. Das kompakte Datagramm und der daraus resultierende reduzierte Bandbreitenbedarf erleichtern somit den Betrieb über eine Fernsteuerverbindung, insbesondere eine drahtlose Verbindung.

Die Erfindung bezieht sich ferner auf ein entsprechendes Verfahren zum Ausgeben einer Hochfrequenz-Wechselspannung des elektrochirurgischen Generators. Zur weiteren Erläuterung der Erzeugung der Hochfrequenz-Wechselspannung mittels des elektrochirurgischen Generators und seines Inverters wird, um unnötige Wiederholungen zu vermeiden, auf die vorstehende Beschreibung des elektrochirurgischen Generators verwiesen, die sinngemäß gilt.

Die Erfindung wird im Folgenden anhand eines vorteilhaften Ausführungsbeispiels näher erläutert. In den Figuren:
- Fig. 1: zeigt ein Blockdiagramm eines chirurgischen Generators gemäß einer beispielhaften Ausführungsform mit einem angeschlossenen elektrochirurgischen Instrument;
- Fig. 2: zeigt ein Blockdiagramm eines kaskadierten Multilevel-Inverters;
- Fig. 3a, b: zeigt eine schematische Darstellung von zwei Beispielen für Datagramme
- Fig. 4: zeigt ein Blockdiagramm einer beispielhaften Ausführungsform einer Referenzsignalbildungseinheit;
- Fig. 5a, b: zeigt ein erstes Beispiel für ein Referenzsignal und Daten des entsprechenden Datagramms;
- Fig. 6a, b: zeigt ein zweites Beispiel für ein Referenzsignal und Daten des entsprechenden Datagramms; und
- Fig. 7:: ein beispielhaftes Flussdiagramm eines Verfahrens zur Bildung des Referenzsignals.

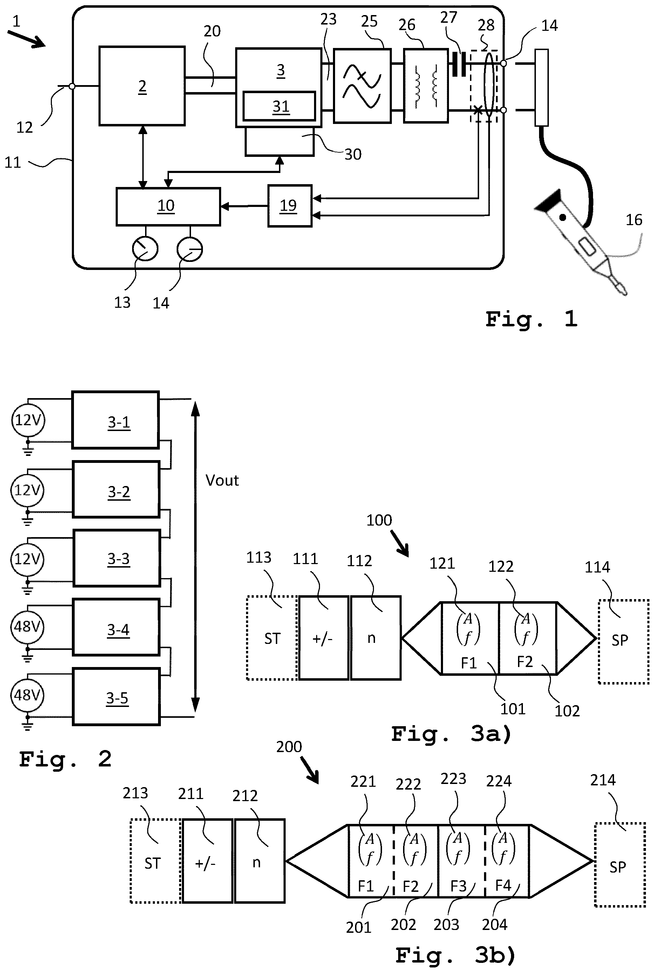

Ein elektrochirurgischer Generator gemäß einer beispielhaften Ausführungsform ist in einem Blockdiagramm in Fig. 1 dargestellt. Der insgesamt mit der Bezugsziffer 1 bezeichnete elektrochirurgische Generator umfasst ein Gehäuse 11, das mit einer Ausgangsbuchse 14 für ein elektrochirurgisches Instrument 16 versehen ist. Dieses ist über ein Hochspannungsanschlusskabel mit der Ausgangsbuchse 14 des elektrochirurgischen Generators 1 verbunden. Für die Stromversorgung des elektrochirurgischen Generators 1 ist ein Netzanschlusskabel 12 vorgesehen, das an ein öffentliches Stromnetz oder eine andere geeignete Stromversorgungseinrichtung angeschlossen werden kann.

Der elektrochirurgische Generator 1 umfasst im Gehäuse 11 ein Netzteil 2, das über die Netzanschlussleitung 12 mit elektrischer Energie versorgt wird (siehe Fig. 1). Das Netzteil 2 umfasst einen Gleichrichter und speist einen Gleichspannungszwischenkreis 20 mit Gleichspannung, der einen Inverter versorgt. Der Inverter 3 erzeugt hochfrequenten Wechselstrom im Hochspannungsbereich von einigen Kilovolt. Die ausgegebene HF-Hochspannung wird über eine Ausgangsleitung 23, ein Tiefpassfilter 25 und über einen Trenntransformator 26, der die Hochspannung hochsetzt, zur Ausgangsbuchse 14 geführt, die einen Reihenkondensator 27 zum Sperren von jeglichem Gleichstrom zur Ausgangsbuchse 14 enthält. Das elektrochirurgische Instrument 16 kann in die Ausgangsbuchse 14 eingesteckt werden. Ausgangsspannung und -strom an der Ausgangsbuchse 14 werden von einem Spannungs- und Stromsensor 28 gemessen, und entsprechende Messsignale werden über eine Rückkopplungsschaltung 19 an die Steuereinheit 10 geliefert. Dies ist in der Technik allgemein bekannt und wird aus Gründen der Kürze nicht weiter erläutert. Der Betrieb des elektrochirurgischen Generators 1 wird von einer Steuereinheit 10 gesteuert, die über Signalleitungen mit der Stromversorgungseinheit 2 und dem Inverter verbunden ist. Die Steuereinheit 10 betreibt den HF-Generator 1 auf der Grundlage von Funktionen, die in einem Speicher der Steuereinheit 10 gespeichert sind. Die Funktionen definieren Betriebseigenschaften und -modi des HF-Generators 1. Die Funktionen können von einem Benutzer mit Hilfe von Eingabegeräten ausgewählt werden, bei denen es sich um herkömmliche Tasten und Knöpfe und/oder eine Touchscreen-Schnittstelle (nicht dargestellt) handeln kann. Zum Beispiel kann die auszugebende Leistung mit einem Leistungswahlschalter 13 und der zu verwendende Modus mit einem Moduswahlschalter 14 ausgewählt werden.

Der Inverter erzeugt eine hochfrequente Wechselspannung im Bereich zwischen 200 kHz und 4 MHz. Der Inverter ist als Multilevel-Inverter 3 ausgeführt. Form, Frequenz, Tastverhältnis und Amplitude der von dem Multilevel-Inverter erzeugten Spannung werden von einer Invertersteuerung 31 auf der Grundlage eines Referenzsignals bestimmt, das von einer Referenzsignalbildungseinheit 30 erzeugt wird, die wiederum von der Steuereinheit 10 in Abhängigkeit von der gewählten Leistung und Betriebsart gesteuert wird.

Der Multilevel-Inverter 3 umfasst eine Vielzahl von Zellen, die in einer kaskadierten Formation angeordnet sind, wie in Fig. 2 gezeigt. Es gibt zwei verschiedene Arten von Zellen, die in zwei Gruppen angeordnet sind, die gruppenweise mit unterschiedlicher Gleichspannung versorgt werden. Die erste Gruppe umfasst die Niederspannungszellen 3-1, 3-2 und 3-3. Sie werden mit einer niedrigeren Gleichspannung versorgt, in der dargestellten Ausführungsform 12 V. Die zweite Gruppe umfasst die Hochspannungszellen 3-4, 3-5, die mit einer höheren Gleichspannung versorgt werden, in der gezeigten Ausführungsform 48 V. Beide Gruppen sind in Reihe geschaltet, so dass die von jeder der Zellen 3-1 bis 3-5 abgegebenen Spannungen zu einer gemeinsamen Ausgangsspannung Vout addiert werden. Die gezeigte Konfiguration kann eine Ausgangsspannung im Bereich von -132 V bis +132 V liefern, was 23 Pegelstufen in 12-V-Schritten entspricht.

In Fig. 3a) und b) sind Beispiele für Datagramme dargestellt. Im ersten Beispiel des in Fig. 3a) gezeigten Datagramms umfasst das Datagramm 100 Datenfelder 101 und 102 für zwei Perioden. Jedes Datenfeld 101 und 102 umfasst einen Datenvektor 121, 122, der jeweils zwei Werte enthält, einen Wert **"A",** der eine Amplitude angibt, und einen anderen Wert "f", der eine Frequenz der jeweiligen Periode angibt. Diese Werte beschreiben also die Eigenschaften der jeweiligen Periode in Bezug auf Amplitude und Frequenz. Ferner enthält das Datagramm 100 ein erstes zusätzliches Datenfeld 111, das die Polarität der ersten Periode angibt, d. h. ob die Schwingung mit einer positiven oder einer negativen Welle beginnt. Darüber hinaus umfasst das Datagramm 100 ein zweites zusätzliches Datenfeld 112, das eine Anzahl "n" von Perioden angibt, für die Datenvektoren in den Datenfeldern 101, 102 enthalten sind, im vorliegenden Fall ist "n" 2. Optional kann das Datagramm 100 auch zusätzliche Datenfelder 113, 114 für ein Startbyte bzw. ein Stoppbyte umfassen.

Die verfügbaren Datenfelder sind in der folgenden Tabelle 1 für ein Datagramm mit Datenfeldern für bis zu 15 Perioden dargestellt:

**Tabelle 1**

| **Parameter** | **Bereich** | **Bits** | **Beschreibung** |
|---|---|---|---|
| *Polarität* | *0 oder 1* | *1* | *0 - pos.* / *1 - neg.* |
| *nPerioden* | *1* ... *15* | *4* | *Anzahl der Perioden* |
| Amplitude0 | 0 ... 127 | 7 | Rel. Ampl. 1. Periode |
| ... | ... | ... | ... |
| Amplitude14 | 0 ... 127 | 7 | Rel. Ampl. 15. Periode |
| Frequenz0 | 0 ... 511 | 9 | Freq. der 1. Periode |
| ... | ... | ... | ... |
| Frequenz14 | 0 ... 511 | 9 | Freq. der 15. Periode |

Die kursiv geschriebenen Zeilen der Tabelle beziehen sich auf zusätzliche Datenfelder. Die anderen Zeilen enthalten den Datenvektor mit einem Amplitudenwert und einem Frequenzwert für jede der bis zu 15 Perioden. Der Amplitudenwert ist ein relativer Amplitudenwert, kein absoluter Wert.

In Fig. 3b) ist ein komplexeres Datagramm 200 dargestellt. Es umfasst vier Datenfelder 201, 202, 203 und 204 für zwei Perioden und getrennt für jede Halbwelle einer der beiden Perioden. Jedes dieser Felder umfasst einen der Datenvektoren 221, 222, 223, 224. Dabei kann für dieselbe Periode ihre positive Halbwelle eine andere Amplitude und/oder Frequenz haben als ihre negative Halbwelle. Weiterhin enthält das Datagramm 200 ähnlich wie das Datagramm 100 ein zusätzliches Datenfeld 211 für die Polarität und ein zusätzliches Datenfeld 212 für die Anzahl der Perioden. Der Wert im zusätzlichen Datenfeld 211 ist ein Indikator für die Polarität der ersten Halbwelle jeder Periode. Darüber hinaus kann das Datagramm 200 auch optionale zusätzliche Datenfelder 213, 214 für ein Startbyte bzw. ein Stoppbyte enthalten.

Die Datenfelder für das Datagramm 200 mit Datenfeldern für jede Halbwelle getrennt sind in der folgenden Tabelle 2 dargestellt:

**Tabelle 2**

| **Parameter** | **Bereich** | **Bits** | **Beschreibung** |
|---|---|---|---|
| *Polarität* | *0 oder 1* | *1* | *0 - pos.* / *1 - neg.* |
| *nPerioden* | *1* ... *15* | *4* | *Anzahl der Perioden* |
| AmplitudeA0 | 0 ... 127 | 7 | Rel. Ampl. 1. Periode 1. Halbwelle |
| ... | ... | ... | ... |
| AmplitudeA14 | 0 ... 127 | 7 | Rel. Ampl. 15. Periode 1. Halbwelle |
| AmplitudeB0 | 0 ... 127 | 7 | Rel. Ampl. 1. Periode 2. Halbwelle |
| ... | ... | ... | ... |
| AmplitudeB14 | 0 ... 127 | 7 | Rel. Ampl. 15. Periode 2. Halbwelle |
| FrequenzA0 | 0 ... 511 | 9 | Freq. der 1. Periode 1. Halbwelle |
| ... | ... | ... | ... |
| FrequenzA14 | 0 ... 511 | 9 | Freq. der 15. Periode 1. Halbwelle |
| FrequenzB0 | 0 ... 511 | 9 | Freq. der 1. Periode 2. Halbwelle |
| ... | | ... | ... |
| FrequenzB14 | 0 ... 511 | 9 | Freq. der 15. Periode 2. Halbwelle |

Ähnlich wie in Tabelle 1 beziehen sich die kursiv geschriebenen Zeilen der Tabelle 2 auf zusätzliche Datenfelder. Die anderen Zeilen enthalten den Datenvektor, der einen Amplitudenwert und einen Frequenzwert für jede Halbwelle jeder der bis zu 15 Perioden umfasst. Der Amplitudenwert ist ein relativer Amplitudenwert, kein absoluter Wert. Es ist erwähnenswert, dass aufgrund dieser Datagramme 200 die Frequenz der zweiten Halbwelle sich von der Frequenz der ersten Halbwelle unterscheiden kann. Dies ist ein wichtiges Merkmal der Erfindung und sorgt für eine größere Flexibilität bei der Gestaltung einer Vielzahl unterschiedlicher Referenzsignale für verschiedene Modi.

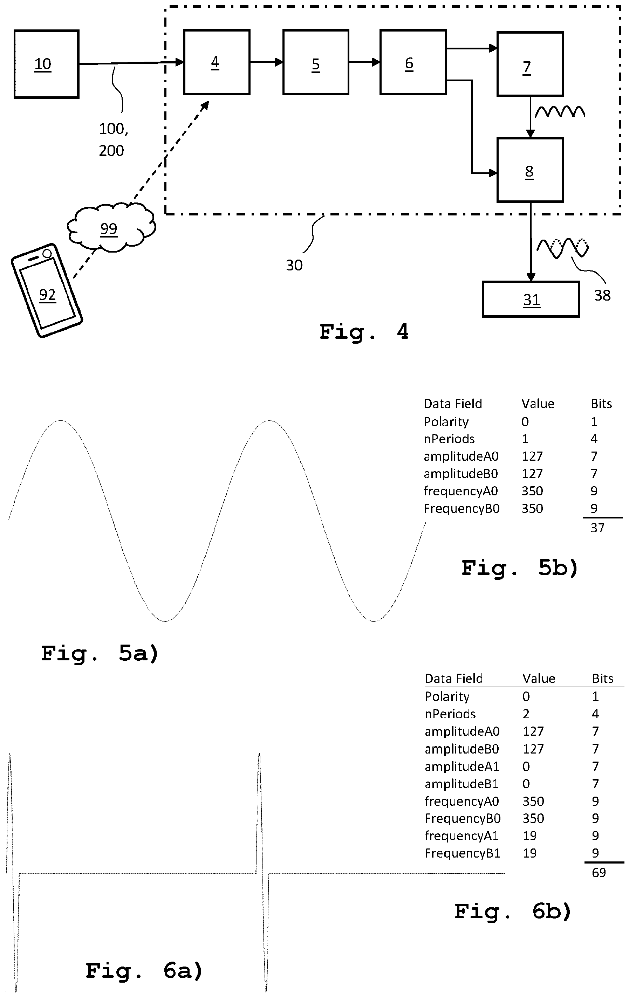

In Fig. 4 dargestellt ist ein Blockdiagramm einer beispielhaften Ausführungsform einer Referenzsignalbildungseinheit 30, die diese Datagramme 100, 200 empfängt, um das Referenzsignal für den Inverter 3 zu bilden. Sie umfasst einen Empfänger 4, der mit der Steuereinheit 10 verbunden ist. Er ist so konfiguriert, dass er das Datagramm 100, 200, welches das zu bildende Referenzsignal beschreibt, von der lokalen Steuereinheit 10 des elektrochirurgischen Generators 1 empfängt.

Optional ist es auch möglich, dass ein solches Datagramm 100, 200 von einem entfernten Gerät 92 über eine, vorzugsweise gesicherte drahtlose Verbindung 99 gesendet wird, wodurch die Fernsteuerung der Referenzsignalbildungseinheit 30 ermöglicht wird. Der geringe Bandbreitenbedarf der Datagramme 100, 200 ist für eine Fernsteuerung, die typischerweise bandbreitenbegrenzt ist, von Vorteil.

Der Empfänger 4 leitet das empfangene Datagramm 100, 200 an einen Decoder 5 weiter. Der Decoder 5 ist so konfiguriert, dass er das Datagramm dekodiert und die Datenvektoren 121, 122, 221, 222, 223, 224 aus den verschiedenen Datenfeldern und ferner die Daten aus den zusätzlichen Datenfeldern 111, 112, 113, 114, 211, 212, 213, 214 extrahiert. Der Decoder 5 liefert die extrahierten Daten an einen Sequencer 6, der so konfiguriert ist, dass er die aus den Datenvektoren extrahierten Daten für jede Periode in eine richtige, durch die Datenfelder definierte Reihenfolge bringt und bei Erreichen des Endes der Sequenz von vorne beginnt, um diese Sequenz immer wieder zu wiederholen. Dies geschieht so lange, bis ein neues Datagramm mit neuen Daten oder ein Datenfragment mit einer Aktualisierung für einige der Datenvektoren empfangen wird, und dann ersetzen diese aktualisierten Datenvektoren die jeweiligen früheren, ohne die Sequenz zu unterbrechen. Die aus den Datenvektoren extrahierten Amplituden- und Frequenzdaten "A" werden einem digital gesteuerten Synthesizer 7 zugeführt, der eine kontinuierliche Schwingung bildet, die eine Folge von Wellen umfasst, wobei Amplitude und Frequenz jeder der Wellen dieser Folge durch die aus den Datenvektoren des Datagramms extrahierten Daten bestimmt werden. Es sei darauf hingewiesen, dass in der in Fig. 4 dargestellten Ausführungsform der digital gesteuerte Synthesizer 7 optional so konfiguriert ist, dass er einen Strom von nur unipolaren, in diesem Fall positiven, Halbwellen liefert. Dies ermöglicht eine doppelte Auflösung bei gleicher Anzahl von Bits.

Um die fehlenden negativen Halbwellen zu schaffen, ist eine schaltbare Inversionsschaltung 8 vorgesehen. Sie ist so konfiguriert, dass sie eine Invertierung jeder zweiten Halbwelle durchführt, wodurch die fehlenden negativen Halbwellen gebildet werden. Zur Steuerung der Inversionsschaltung 8 wird ein Signal bezüglich der Polarität aus einem zusätzlichen Datenfeld 111, 211 entnommen. Wenn die extrahierten Polaritätsdaten eine positive Polarität verlangen, werden die erste und jede folgende ungerade Halbwelle nicht invertiert und die Invertierung wird an der zweiten und jeder folgenden geraden Halbwelle durchgeführt, wodurch Vollwellen mit einer positiven ersten Halbwelle entstehen. Umgekehrt, wenn die extrahierten Polaritätsdaten eine negative Polarität erfordern, wird die Invertierung an der ersten und jeder folgenden ungeraden Halbwelle durchgeführt, wodurch Vollwellen mit negativer erster Halbwelle erzeugt werden. Damit ist die Bildung des Vollwellen-Referenzsignals 38 abgeschlossen, das von der Referenzsignalbildungseinheit 30 ausgegeben und der Inverter-Steuerung 31 zur Verarbeitung durch den Multilevel-Inverter 3 zugeführt wird.

Ein Beispiel für ein Referenzsignal, das auf diese Weise von der Referenzsignalbildungseinheit 30 gebildet wird, ist in Fig. 5a dargestellt. Das Referenzsignal ist ein nicht-komplexes Signal. Im vorliegenden Beispiel handelt es sich um eine sinusförmige Welle, die für einen "Pure Cut"-Modus des elektrochirurgischen Generators 1 geeignet ist. Die Datenwerte und Parameter, die für die verschiedenen Datenfelder des Datagramms verwendet werden, sind in Fig. 5b dargestellt. Außerdem ist die Bitlänge für jedes der Datenfelder angegeben. Die gesamte Datenmenge, die für die Steuerung des Multilevel-Inverters 3 durch die Definition des Referenzsignals mit dem Datagramm erforderlich ist, beträgt nur 37 Bit. Dieses Datagramm muss nur einmal übertragen werden, und die Einheit 30, die das Referenzsignal bildet, erzeugt ein kontinuierliches Referenzsignal mit der Form, Amplitude und Frequenz wie durch das Datagramm definiert. Dies steht in krassem Gegensatz zu den großen Datenmengen, die für die herkömmliche Steuerung des Multilevel-Inverters 3 erforderlich sind.

Ein Beispiel für ein komplexeres Referenzsignal ist in Fig. 6a dargestellt. Dieses Referenzsignal ist komplexer und umfasst zwei Perioden, für die Halbwellen definiert sind. Es verwendet also ein Datagramm wie das in Fig. 3b gezeigte Datagramm 200. Die Frequenz der ersten Periode ist recht hoch (350 Hz) und die Frequenz der zweiten Periode ist viel niedriger (19 Hz). Zusammen mit einer hohen Amplitude für die erste Periode und einer niedrigen, genauer gesagt null, Amplitude für die zweite Periode wird ein nadelähnliches Referenzsignal mit einem Tastverhältnis von etwa 5,5 % erzeugt, das sich gut für einen "Spray- und Koagulationsmodus" des elektrochirurgischen Generators 1 eignet. Wie dieses Beispiel zeigt, können selbst komplexe Referenzsignale mit hohen Tastverhältnissen mit einem Minimum an Daten, im vorliegenden Beispiel nur 69 Bit, gebildet werden. Dies steht wiederum im krassen Gegensatz zu den hohen Datenmengen, die für die konventionelle Steuerung des Multilevel-Inverters 3 erforderlich sind.

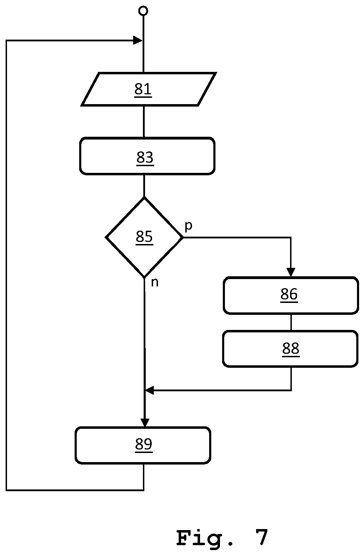

In Fig. 7 ist ein beispielhaftes Flussdiagramm für ein Verfahren zur Bildung des Referenzsignals für die Steuerung des Multilevel-Inverters 3 dargestellt. Nach dem Empfang eines Datagramms 100 in Schritt 81 wird in Schritt 83 ein Referenzsignal gebildet, wie oben beschrieben. In vielen Fällen wird das durch das Datagramm 100 definierte Referenzsignal direkt (über den Zweig "n") zur Steuerung des Inverters 3 in Schritt 89 verwendet, um Form, Frequenz und Amplitude der vom Multilevel-Inverter 3 erzeugten Hochfrequenz-Wechselspannung zu definieren. Wenn jedoch eine Überprüfung des Empfangs eines Datenfragments in Schritt 85 ergibt, dass ein solches Datenfragment empfangen wurde, verzweigt der Arbeitsablauf (über Zweig "p"), um in Schritt 86 eine Extraktion von Datenvektoren durchzuführen, die in den Datenfeldern enthalten sind, die in dem besagten Datenfragment enthalten sind. Anschließend werden die somit extrahierten Datenvektoren die entsprechenden vorherigen Datenvektoren ersetzen und ein aktualisiertes Referenzsignal wird in Schritt 88 gebildet. Schließlich wird in Schritt 89 der Multilevel-Inverter 3 auf der Grundlage des Referenzsignals gesteuert, falls zutreffend einschließlich etwaiger Aktualisierungen durch die Datenfragmente, um die hochfrequente Wechselspannung zu erzeugen, deren Form, Frequenz und Amplitude definiert sind durch die zur Bildung des Referenzsignals verwendeten Datagramme 100.

## Patentansprüche

1. Elektrochirurgischer Generator zur Ausgabe einer Hochfrequenz-Wechselspannung an ein elektrochirurgisches Instrument (16), umfassend eine Steuereinheit (10) und einen Inverter (3) für Hochspannung, der eine Hochfrequenz-Wechselspannung mit variabler Frequenz und Amplitude erzeugt, die einer Ausgangsbuchse (14) zum Anschluss des elektrochirurgischen Instruments (16) zugeführt wird, wobei der Inverter ein Multilevel-Inverter (3) ist, der durch ein Referenzsignal gesteuert wird, das die Form der erzeugten Hochfrequenz-Wechselspannung definiert, **dadurch gekennzeichnet, dass** das Referenzsignal durch ein Datagramm (100, 200) definiert ist, wobei das Datagramm (100, 200) eine endliche Reihe von aufeinanderfolgenden Amplituden- und Frequenzdatenvektoren für eine definierte Anzahl von Perioden umfasst.

2. Elektrochirurgischer Generator nach Anspruch 1, wobei das Referenzsignal von einer Referenzsignalbildungseinheit (30) bereitgestellt ist.

3. Elektrochirurgischer Generator nach Anspruch 2, wobei die Referenzsignalbildungseinheit (30) umfasst:
- einen Empfänger (4), der ausgebildet ist zum Empfangen eines Datensatzes mit dem Datagramm (100, 200),
- einen Decoder (5), der dazu ausgebildet ist, den vom Empfänger (4) empfangenen Datensatz zu dekodieren und die Datenvektoren für jede der Perioden zu extrahieren,
- einen Sequenzer (6), der dazu ausgebildet ist, Signale für Amplitude und Frequenz aus dem extrahierten Datenvektor für jede Periode in einer durch die Datenfelder definierten Folge auszugeben und diese Folge zu wiederholen, und
- einen Synthesizer (7), der so konfiguriert ist, dass er für jeden der Datenvektoren eine Oszillationswelle mit der Amplitude und Frequenz gemäß dem jeweiligen Datenvektor bildet, wobei eine Reihe der Oszillationswellen das Referenzsignal bilden, das dem Inverter (3) zugeführt wird.

4. Elektrochirurgischer Generator nach einem der vorhergehenden Ansprüche, wobei das Datagramm (100, 200) eine Vielzahl von Datenfeldern (101, 102; 201, 202, 203, 204) umfasst, ein Datenfeld für jede Periode, wobei jedes Datenfeld einen der Datenvektoren umfasst mit Werten, die für die Amplitude und die Frequenz einer jeweiligen der definierten Anzahl von Perioden indikativ sind.

5. Elektrochirurgischer Generator nach einem der vorhergehenden Ansprüche, wobei das Datagramm (100, 200) ferner ein erstes zusätzliches Datenfeld (111, 211) umfasst, das für eine Polarität, insbesondere die Polarität einer ersten Halbwelle einer ersten Periode, indikativ ist.

6. Elektrochirurgischer Generator nach einem der vorhergehenden Ansprüche, wobei das Datagramm (100, 200) ferner ein zweites zusätzliches Datenfeld (112, 113) umfasst, das indikativ ist für die Anzahl der Perioden.

7. Elektrochirurgischer Generator nach einem der vorhergehenden Ansprüche, wobei das Datagramm (100, 200) zusätzliche Datenfelder (113, 114; 213, 214) umfasst, die indikativ sind für auf Start- und Stoppinformationen, insbesondere Start- und Stoppbytes.

8. Elektrochirurgischer Generator nach einem der Ansprüche 3 bis 7, wobei der Synthesizer (7) zur Bildung von Halbwellenschwingungen ausgebildet ist, und die Perioden im Datagramm (100, 200) sich auf die Dauer der Halbwellenschwingung beziehen.

9. Elektrochirurgischer Generator nach dem vorhergehenden Anspruch, wobei der Synthesizer (7) so konfiguriert ist, dass er nur unipolare Halbwellen bildet, insbesondere positive Halbwellen.

10. Elektrochirurgischer Generator nach dem vorhergehenden Anspruch, wobei eine Inversionsschaltung (8) mit dem Synthesizer (7) gekoppelt ist und so konfiguriert ist, dass sie jede zweite vom Synthesizer (7) abgegebene Halbwelle invertiert.

11. Elektrochirurgischer Generator nach dem vorhergehenden Anspruch, wobei die Inversionsschaltung (8) eine schaltbare Inversionsschaltung ist, die in Abhängigkeit von einem Auswahlsignal schaltbar ist, ob gerade oder ungerade Halbwellen zu invertieren sind.

12. Elektrochirurgischer Generator nach dem vorhergehenden Anspruch, wobei das Auswahlsignal automatisch entsprechend einem ersten zusätzlichen Datenfeld (111, 211), das die Polarität angibt, eingestellt wird.

13. Elektrochirurgischer Generator nach einem der Ansprüche 3 bis 12, wobei der Empfänger (4) ferner dazu ausgebildet ist er ein Datagrammfragment zu empfangen, wobei das Datagrammfragment eine eingeschränkte Anzahl der Datenfelder (101, 102; 201, 202, 203, 204) mit geänderten Amplituden- und Frequenzdatenvektoren und eine Kennung für die betreffende Periode umfasst, wobei die eingeschränkte Anzahl eine oder mehrere, aber weniger ist als die definierte Anzahl von Perioden.

14. Elektrochirurgischer Generator nach dem vorhergehenden Anspruch, wobei der Decoder dazu ausgebildet ist, die Datenvektoren und Identifikatoren zu extrahieren, und der Sequenzer ausgebildet ist, selektiv für die betreffende Periode die Ausgangssignale für Amplitude und Frequenz zu ersetzen durch diejenigen, die in dem dekodierten Datagrammfragment empfangen wurden.

15. Elektrochirurgischer Generator nach einem der vorhergehenden Ansprüche, wobei das Datagramm (100, 200) so verdichtet ist, dass die Bitlänge des Amplituden- und/oder Frequenzwertes im Datenvektor begrenzt ist auf die Anzahl der Pegel des Multilevel-Inverters bzw. den Frequenzbereich.

16. Elektrochirurgischer Generator nach einem der Ansprüche 3 bis 15, wobei der Empfänger (4) mit der Steuereinheit (10) zum Empfang des Datagramms (100, 200) gekoppelt ist und/oder konfiguriert ist zum Empfang des Datagramms (100, 200) von einer entfernten Steuereinheit (92).

17. Verfahren zum Ausgeben einer Hochfrequenz-Wechselspannung mittels eines elektrochirurgischen Generators, die zur Ausgabe an ein elektrochirurgisches Instrument (16) bestimmt ist, wobei der elektrochirurgische Generator eine Steuereinheit (10) und einen Inverter (3) für Hochspannung umfasst, der eine hochfrequente Wechselspannung mit variabler Frequenz und Amplitude erzeugt, die einer Ausgangsbuchse (14) zum Anschluss des elektrochirurgischen Instruments (16) zugeführt wird, wobei der Inverter ein Multilevel-Inverter (3) ist; das Verfahren umfasst: Steuern (89) des Multilevel-Inverters (3) durch ein Referenzsignal, das die Form der erzeugten Hochfrequenz-Wechselspannung definiert; 30 und ist **gekennzeichnet durch**: Bilden (83) des Referenzsignals gemäß einem Datagramm (100, 200), wobei das das Referenzsignal definierende Datagramm (100, 200) eine endliche Reihe von sequenzierten Amplituden- und Frequenzdatenvektoren für eine definierte Anzahl von Perioden umfasst

18. Verfahren nach Anspruch 17, wobei das Referenzsignal nach einem der Ansprüche 2 bis 16 gebildet wird.

## Claims

1. Electrosurgical generator for outputting a radiofrequency AC voltage to an electrosurgical instrument (16), comprising a control unit (10) and a high-voltage inverter (3) that creates a radiofrequency AC voltage of variable frequency and amplitude that is supplied to an output socket (14) for connecting the electrosurgical instrument (16),
wherein the inverter is a multilevel inverter (3) that is controlled by a reference signal which defines the shape of the created radiofrequency AC voltage, **characterized in that** the reference signal is defined by a datagram (100, 200), wherein the datagram (100, 200) comprises a finite series of consecutive amplitude and frequency data vectors for a defined number of periods.

2. Electrosurgical generator according to Claim 1, wherein the reference signal is provided by a reference signal forming unit (30).

3. Electrosurgical generator according to Claim 2, wherein the reference signal forming unit (30) comprises:
- a receiver (4) that is designed to receive a data set with the datagram (100, 200),
- a decoder (5) that is designed to decode the data set received by the receiver (4) and extract the data vectors for each of the periods,
- a sequencer (6) that is designed to output signals for the amplitude and frequency from the extracted data vector for each period in a sequence which is defined by the data fields and repeat this sequence, and
- a synthesizer (7) that is configured to form for each of the data vectors an oscillation wave with the amplitude and frequency according to the respective data vector, wherein a series of oscillation waves form the reference signal that is supplied to the inverter (3).

4. Electrosurgical generator according to any of the preceding claims, wherein the datagram (100, 200) comprises a multiplicity of data fields (101, 102; 201, 202, 203, 204), one data field for each period, wherein each data field comprises one of the data vectors with values that are indicative of the amplitude and the frequency of a respective one of the defined number of periods.

5. Electrosurgical generator according to any of the preceding claims, wherein the datagram (100, 200) further comprises a first additional data field (111, 211) that is indicative of a polarity, in particular the polarity of a first half wave of a first period.

6. Electrosurgical generator according to any of the preceding claims, wherein the datagram (100, 200) further comprises a second additional data field (112, 113) that is indicative of the number of periods.

7. Electrosurgical generator according to any of the preceding claims, wherein the datagram (100, 200) comprises additional data fields (113, 114; 213, 214) that are indicative of start and stop information, in particular start and stop bytes.

8. Electrosurgical generator according to any of Claims 3 to 7, wherein the synthesizer (7) is designed to form half-wave vibrations, and the periods in the datagram (100, 200) refer to the duration of the half-wave vibration.

9. Electrosurgical generator according to the preceding claim, wherein the synthesizer (7) is configured such that it forms only unipolar half waves, in particular positive half waves.

10. Electrosurgical generator according to the preceding claim, wherein an inversion circuit (8) is coupled to the synthesizer (7) and configured such that it inverts every second half wave emitted by the synthesizer (7).

11. Electrosurgical generator according to the preceding claim, wherein the inversion circuit (8) is a switchable inversion circuit that is switchable depending on a selection signal in relation to whether even or odd half waves should be inverted.

12. Electrosurgical generator according to the preceding claim, wherein the selection signal is automatically set in accordance with a first additional data field (111, 211) that is indicative of the polarity.

13. Electrosurgical generator according to any of Claims 3 to 12, wherein the receiver (4) is further designed to receive a datagram fragment, wherein the datagram fragment comprises a limited number of data fields (101, 102; 201, 202, 203, 204) with modified amplitude and frequency data vectors and an identifier for the period in question, wherein the limited number is one or more periods, but less than the defined number of periods.

14. Electrosurgical generator according to the preceding claim, wherein the decoder is designed to extract the data vectors and identifiers, and the sequencer is designed to selectively replace the output signals for amplitude and frequency for the period in question with those received in the decoded datagram fragment.

15. Electrosurgical generator according to any of the preceding claims, wherein the datagram (100, 200) is compressed such that the bit length of the amplitude and/or frequency value in the data vector is limited to the number of levels in the multilevel inverter or to the frequency range.

16. Electrosurgical generator according to any of Claims 3 to 15, wherein the receiver (4) is coupled to the control unit (10) for receiving the datagram (100, 200) and/or configured to receive the datagram (100, 200) from a remote control unit (92).

17. Method for outputting a radiofrequency AC voltage by means of an electrosurgical generator, which is intended for output to an electrosurgical instrument (16), wherein the electrosurgical generator comprises a control unit (10) and a high-voltage inverter (3) that creates a radiofrequency AC voltage of variable frequency and amplitude that is supplied to an output socket (14) for connecting the electrosurgical instrument (16), wherein the inverter is a multilevel inverter (3); the method comprises:
controlling (89) the multilevel inverter (3) by means of a reference signal which defines the shape of the created radiofrequency AC voltage;
and is **characterized by**: forming (83) the reference signal according to a datagram (100, 200), wherein the datagram (100, 200) that defines the reference signal comprises a finite series of sequenced amplitude and frequency data vectors for a defined number of periods.

18. Method according to Claim 17, wherein the reference signal is formed according to any of Claims 2 to 16.

## Revendications

1. Générateur électrochirurgical destiné à délivrer une tension alternative à haute fréquence à un instrument électrochirurgical (16), comprenant une unité de commande (10) et un onduleur (3) pour haute tension qui génère une tension alternative à haute fréquence de fréquence et d'amplitude variables, qui est acheminée à une prise de sortie (14) servant au raccordement de l'instrument électrochirurgical (16),
l'onduleur étant un onduleur multiniveau (3) qui est commandé par un signal de référence, lequel définit la forme de la tension alternative à haute fréquence générée, **caractérisé en ce que** le signal de référence est défini par un datagramme (100, 200), le datagramme (100, 200) comprenant une série finie de vecteurs de données d'amplitude et de fréquence consécutifs pour un nombre défini de périodes.

2. Générateur électrochirurgical selon la revendication 1, le signal de référence étant fourni par une unité de formation de signal de référence (30).

3. Générateur électrochirurgical selon la revendication 2, l'unité de formation de signal de référence (30) comprenant :
- un récepteur (4), qui est configuré pour recevoir un jeu de données avec le datagramme (100, 200),
- un décodeur (5), qui est configuré pour décoder le jeu de données reçu par le récepteur (4) et pour extraire les vecteurs de données pour chacune des périodes,
- un séquenceur (6), qui est configuré pour délivrer en sortie des signaux pour l'amplitude et la fréquence à partir du vecteur de données extrait pour chaque période dans une séquence définie par les champs de données et pour répéter cette séquence, et
- un synthétiseur (7), qui est configuré de telle sorte qu'il forme, pour chacun des vecteurs de données, une onde d'oscillation ayant l'amplitude et la fréquence selon le vecteur de données respectif, une série des ondes d'oscillation formant le signal de référence qui est acheminé à l'onduleur (3).

4. Générateur électrochirurgical selon l'une des revendications précédentes, le datagramme (100, 200) comprenant une pluralité de champs de données (101, 102 ; 201, 202, 203, 204), un champ de données pour chaque période, chaque champ de données comprenant l'un des vecteurs de données ayant des valeurs qui sont indicatives de l'amplitude et de la fréquence de l'une respective du nombre défini de périodes.

5. Générateur électrochirurgical selon l'une des revendications précédentes, le datagramme (100, 200) comprenant en outre un premier champ de données supplémentaire (111, 211) qui est indicatif d'une polarité, notamment la polarité d'une première demi-onde d'une première période.

6. Générateur électrochirurgical selon l'une des revendications précédentes, le datagramme (100, 200) comprenant en outre un deuxième champ de données supplémentaire (112, 113) qui est indicatif du nombre de périodes.

7. Générateur électrochirurgical selon l'une des revendications précédentes, le datagramme (100, 200) comprenant des champs de données supplémentaires (113, 114 ; 213, 214) qui sont indicatifs d'informations de départ et d'arrêt, notamment des octets de départ et d'arrêt.

8. Générateur électrochirurgical selon l'une des revendications 3 à 7, le synthétiseur (7) étant configuré pour former des oscillations de demi-onde, et les périodes dans le datagramme (100, 200) se rapportant à la durée de l'oscillation de demi-onde.

9. Générateur électrochirurgical selon la revendication précédente, le synthétiseur (7) étant configuré de telle sorte qu'il forme uniquement des demi-ondes unipolaires, notamment des demi-ondes positives.

10. Générateur électrochirurgical selon la revendication précédente, un circuit d'inversion (8) étant couplé au synthétiseur (7) et étant configuré de telle sorte qu'il inverse chaque deuxième demi-onde délivrée par le synthétiseur (7).

11. Générateur électrochirurgical selon la revendication précédente, le circuit d'inversion (8) étant un circuit d'inversion commutable qui peut être commuté en fonction d'un signal de sélection afin de définir si des demi-ondes paires ou impaires doivent être inversées.

12. Générateur électrochirurgical selon la revendication précédente, le signal de sélection étant réglé automatiquement conformément à un premier champ de données supplémentaire (111, 211) qui indique la polarité.

13. Générateur électrochirurgical selon l'une des revendications 3 à 12, le récepteur (4) étant en outre configuré pour recevoir un fragment de datagramme, le fragment de datagramme comprenant un nombre restreint des champs de données (101, 102 ; 201, 202, 203, 204) ayant des vecteurs de données d'amplitude et de fréquence modifiés et un identifiant pour la période concernée, le nombre restreint étant une ou plusieurs, mais étant inférieur au nombre défini de périodes.

14. Générateur électrochirurgical selon la revendication précédente, le décodeur étant configuré pour extraire les vecteurs de données et les identificateurs, et le séquenceur étant configuré pour remplacer sélectivement, pour la période concernée, les signaux de sortie pour l'amplitude et la fréquence par ceux qui ont été reçus dans le fragment de datagramme décodé.

15. Générateur électrochirurgical selon l'une des revendications précédentes, le datagramme (100, 200) étant compressé de telle sorte que la longueur binaire de la valeur d'amplitude et/ou de fréquence dans le vecteur de données est limitée au nombre de niveaux de l'onduleur multiniveau ou à la plage de fréquences.

16. Générateur électrochirurgical selon l'une des revendications 3 à 15, le récepteur (4) étant couplé à l'unité de commande (10) pour recevoir le datagramme (100, 200) et/ou est configuré pour recevoir le datagramme (100, 200) d'une unité de commande distante (92).

17. Procédé pour délivrer une tension alternative à haute fréquence au moyen d'un générateur électrochirurgical, laquelle est destinée à être délivrée à un instrument électrochirurgical (16), le générateur électrochirurgical comprenant une unité de commande (10) et un onduleur (3) pour haute tension, lequel génère une tension alternative à haute fréquence de fréquence et d'amplitude variables qui est acheminée à une prise de sortie (14) servant au raccordement de l'instrument électrochirurgical (16),
l'onduleur étant un onduleur multiniveau (3) ; le procédé comprenant :
commande (89) de l'onduleur multiniveau (3) par un signal de référence qui définit la forme de la tension alternative à haute fréquence générée ;
et étant **caractérisé par** : formation (83) du signal de référence conformément à un datagramme (100, 200), le datagramme (100, 200) qui définit le signal de référence comprenant une série finie de vecteurs de données d'amplitude et de fréquence séquencés pour un nombre défini de périodes.

18. Procédé selon la revendication 17, le signal de référence étant formé selon l'une des revendications 2 à 16.
